# EUROPEAN PATENT SPECIFICATION

(11) **EP 3 598 958 B1**
(45) Date of publication and mention of the grant of the patent: **02.03.2022**
(21) Application number: 19187718.2
(22) Date of filing: 23.07.2019
(51) Int. Cl.: A61F 5/00

(54) **APPARATUS FOR ANCHORING A GASTROINTESTINAL IMPLANT**
VORRICHTUNG ZUR VERANKERUNG EINES GASTROINTESTINALEN IMPLANTATS
APPAREIL D'ANCRAGE D'UN IMPLANT GASTRO-INTESTINAL

(30) Priority: 25.07.2018 US 201816044817
(43) Date of publication of application: 29.01.2020
(73) Proprietor: USGI Medical Inc, San Clemente CA 92673 (US)
(72) Inventor: EARLEY, Christopher James, Laguna Niguel, CA 92677 (US); MAAHS, Tracy D., Lake Forest, CA 92630 (US)
(74) Representative: Schaumburg und Partner Patentanwälte mbB

(56) References cited:
- EP-A1- 3 730 099
- WO-A2-2011/011629
- US-A1- 2005 251 208
- US-A1- 2013 217 957

## Description

### BACKGROUND OF THE INVENTION

Gastrointestinal implants may be permanently or semi-permanently implanted into the gastrointestinal tract to treat obesity, diabetes, and other conditions. Gastrointestinal implants may have various forms, such as space occupying devices and bypass sleeves. One form of gastrointestinal implant is a gastrointestinal sleeve. A gastrointestinal sleeve is generally a thin walled flexible sleeve placed into the gastrointestinal tract to limit the absorption of food. The gastrointestinal implant may have various forms of attachments to prevent the sleeve from moving out of position. However, keeping gastrointestinal sleeves and other gastrointestinal implants in place in the gastrointestinal tract has presented various challenges. The stomach and intestines are subject to the movement of food or chyme past or through the gastrointestinal sleeve or implant. Normal muscular contractions of gastrointestinal tract tissues around the gastrointestinal sleeve or implant may exert forces tending to displace it. The gastrointestinal sleeve or implant may also be exposed to stomach acids and gas bubbles, which can contribute to undesirable movement of the gastrointestinal sleeve or implant. Accordingly, improved designs and techniques are needed to secure gastrointestinal sleeves and implants in place.

Tissue anchors may be used to hold or fix a gastrointestinal implant in place. Anchor pairs placed in gastric tissue and drawn towards each other to form a plication provide a fixation location for a gastrointestinal implant, to prevent migration of the gastrointestinal implant. For example, anchor pairs placed in the body or antrum of the stomach can serve as a fixation point for a gastric implant positioned in the duodenum or at the pylorus. A tether may extend from the gastric implant to the implanted anchor pairs, in various forms.

Using needles to create tissue anchor points in the stomach is known in the art, and an application of such to reduce stomach volume is described in US patent application publication US 2013/0217957 A1.

Implantable medical devices are also known in the art, such as the international patent application publication WO 2011/011629 A2 discussing retrieving implantable medical devices.

### BRIEF DESCRIPTION OF THE DRAWINGS

In the drawings, the same reference number indicates the same element in each of the views.
Fig. 1 is a schematic view of a tissue anchor pair tethered to a gastrointestinal implant.
Fig. 2 is an enlarged detail view of the tissue anchor pair and tether attachment shown in Fig. 1.
Figs. 3A and 3B are schematic views of a tether loop placed over a needle and a catheter body during anchor placement.
Fig. 4 is a schematic view of multiple tethers attached to multiple anchor pairs.
Fig. 5 is an enlarged detail view of the multiple anchor pairs shown in Fig. 4.
Fig. 6 is a view showing the gastrointestinal implant attached to tissue anchors positioned in the duodenum.
Fig. 7 is a side view of a tether with a looped attachment.
Fig. 8 is a schematic view of one anchor inside of the gastrointestinal implant while the other anchor of the anchor pair is outside of the gastrointestinal implant.
Fig. 9 is a schematic view of a gastric tissue fold pulled directly through an opening in the side wall of the gastrointestinal implant.
Fig. 10 is a schematic view of an implanted anchor pair with an extended looped section of suture attaching to a feature on the gastrointestinal implant.
Fig. 11A is a schematic view of a loop having a tab adapted to be grasped by a surgical grasping tool.
Fig. 11B is a schematic view of a surgical grasping tool grasping the tab shown in Fig. 11A.
Figs. 12A to 12C are schematic views of a clasp having a pivoting arm.
Fig. 13 is a schematic view of a clasp having a flex arm.
Figs. 14A to 14C are schematic views of a gastrointestinal implant having a straight tee bar.
Fig. 15 is a schematic view of a gastrointestinal implant having a V-shape or a cone shape bar.
Fig. 16 is a schematic view of a loop to loop attachment used to attach a gastrointestinal implant to an implanted tissue anchor pair.
Fig. 17 is a schematic view of an anchor pair integrated directly into a gastrointestinal implant.
Figs. 18A to 18I are schematic views showing a sequence of steps for using the gastrointestinal implant of Fig. 17.
Fig. 19 is a schematic view of a clasp pair assembly.
Fig. 20 is a schematic view of the clasp pair assembly of Fig. 19 attaching a gastrointestinal implant to a tissue anchor pair.

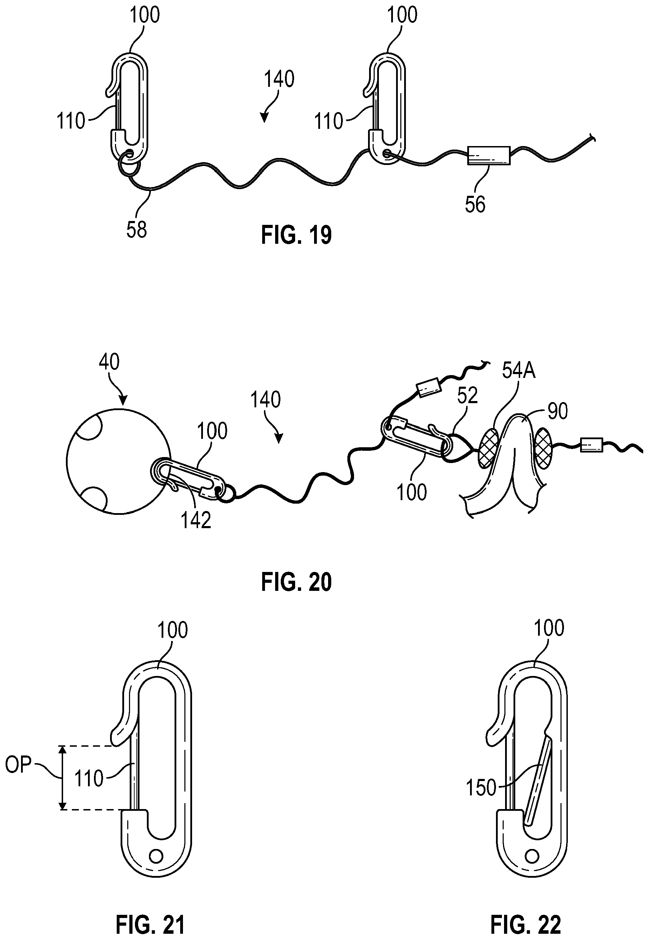

Figs. 20-27 are schematic views of alternative clasp designs.

### DETAILED DESCRIPTION

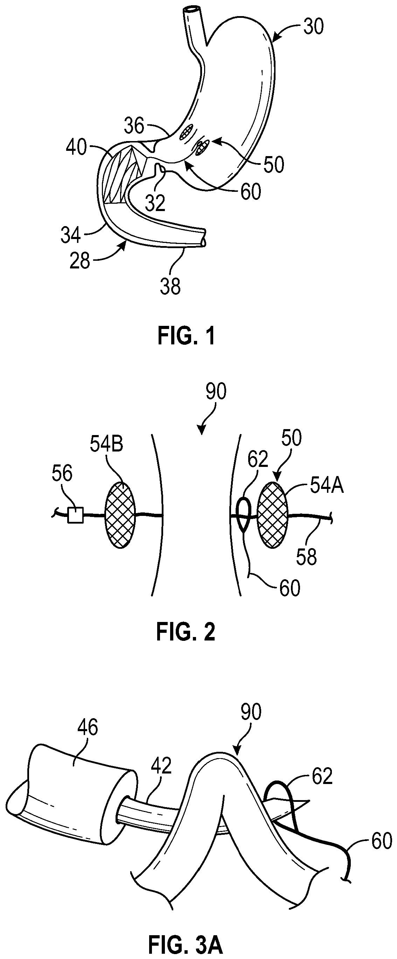

Fig. 1 shows a tether 60 from a gastrointestinal implant in the duodenum 34 leads to an anchor pair 50, where a loop 52 on the end of the tether is placed over a tissue anchor 54A before using a cinch 56 for cinching the tissue anchor 54A into a fixed position on a length of suture 58. The tether 60 can be a wire or suture.

Fig. 2 shows a detailed view of the anchor pair 50 and the tether 60. After the tether loop 62 is placed over the anchor 54A, the anchor pair 50 is then cinched, trapping the tether loop 62 between the anchor 54A and the tissue plication 90.

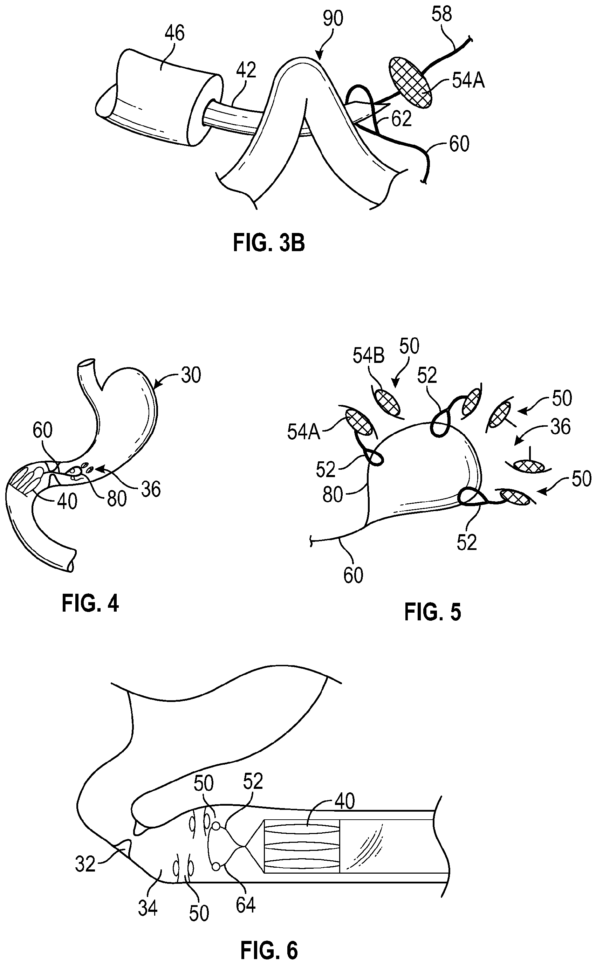

As shown in Figs. 3A and 3B, the tether loop 62 may alternatively be placed over the needle 42 and catheter 46 during anchor placement, but before deployment of the distal anchor 54A. This option may provide for a smaller tether loop diameter to minimize the loop from inadvertently slipping back over the anchor.

As shown in Figs. 4 and 5, the loop 52 used for attachment may alternatively be a feature of the implanted anchor pairs 50 and the tether 60 of the implant 40 may pass through the loop 52 as means of attachment. The tether 60 of the implant 40 may have an enlarged tether end loop or ring 80 providing attachment to two or more tissue anchor pairs 50, as shown in Figs. 4 and 5. The anchors forming the anchor pairs may be delivered and placed using known techniques, for example as described in U.S. Patent No. 7,736,374. The tissue anchors 54A, 54B may be designed as described in U.S. Patent No. 8,870,916.

One or more tethers 60 may be attached to one or more anchor pairs 50. Figs. 4 and 5 show three anchor pairs 50 in the antrum 36 of the stomach 30. Anchor pairs 50 may optionally be placed directly into the duodenum 34 or anywhere along the gastrointestinal tract 28 or in the stomach 30.

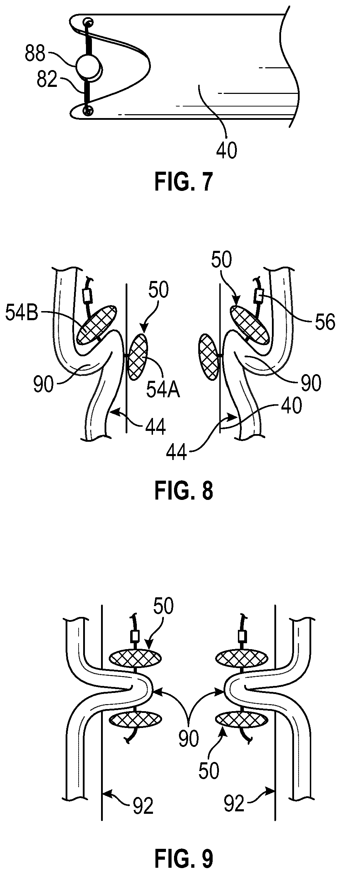

As shown in Fig. 6, the tether 60 may be an extended member 64 from the gastrointestinal implant 40. The attachment feature may be immediately adjacent to or closer to the implant 40. For example, the implant 40 may include an implant ring 88 on a wire or suture 82 threaded through the implant, as shown in Fig. 7. The implant ring 88 may include loops or other attachment mechanisms for interfacing with the anchor pairs 50.

Holes or loops for attaching an implant 40 to the anchor pairs 50 may also be placed directly into the implant 40. For example, one anchor 54A may be on the inside of the implant 40 while the other anchor 54B of the anchor pair 50 is on the outside, as shown in Fig. 8, where the tissue folds 90 are formed in the walls 44 of the gastrointestinal tract.

In another embodiment, a gastric tissue fold 90 is pulled directly through the side wall 92 of the implant 40, securing the implant 40 underneath using tissue folds 90 or the anchor pair 50, or both, as shown in Fig. 9. Tissue folds 90 may formed using known tools and techniques, for example as described in U.S. Patent No. 8,828,027.

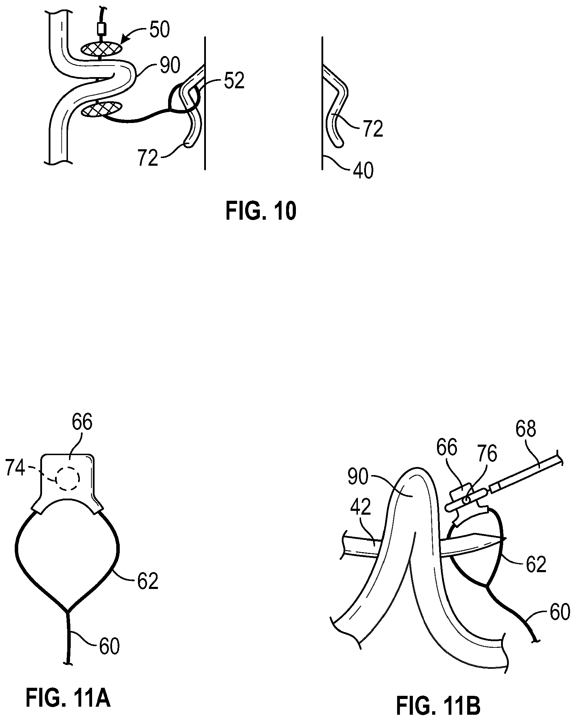

In another embodiment, loops of wire or suture 58 of the anchor pair 50 may attach directly to prongs, hooks, or other types of protrusions 72 on the gastrointestinal implant 40, as shown in Fig. 10.

In the example of Fig. 10, the anchor pair 50 or pairs may be applied to the tissue without cinching, and then the implant 40 may be moved into its position and attached to the loop 52 of the anchor pair 50, and then the anchors 54A, 54B may be cinched, pulling the loop 52 towards the anchor pair 50 and tightening the implant 40 into place. The cinch 56 is designed to slide relatively freely along the wire or suture in a first direction, but cannot move or slide in the opposite direction.

Similarly, an extension of the tether 60 leading from the anchor pair 50 and to the implant 40 may also originate in the antrum 36 and lead into the small intestine, where a loop or similar attachment mechanism may be used for fixing the implant 40.

Referring to Figs. 3A and 3B the placement of the tether loop 62 over the needle 42 and catheter 46 during anchor deployment, the tether loop 62 may include additional features for easing the ability to accurately place the loop over the needle. For example, the loop may include a tab 66 adapted to be grasped by a grasping tool 68, as shown in Figs. 11A and 11B. The tab 66 allows the surgeon to grasp the end of the loop 52 without obstructing the opening diameter of the loop, while also helping to avoid inadvertent damage to the loop by the grasping tool 68. The molded shape or tab may help to maintain the opening of the loop 52 and prevent the loop from collapsing, as may occur with a loop made of suture material such as braided polyester.

In Figs. 11A and 11B the tab 66 is shown at the distal end of the loop 52. However, the tab 66 may be located anywhere along the loop 52 of further down on the tether 60 away from the loop 52. The tab 66 may also have other shapes. The tab 66 may be specifically designed to cooperate with the grasping tool 68. For example, the tab 66 may have a through opening 74, and the grasping tool 68 may have a projection 76 which is moved into and/or through the opening 74, to securely grasp the tab 66. The tab 66 may be silicone molded over a braided polyester suture, or a polypropylene monfilament.

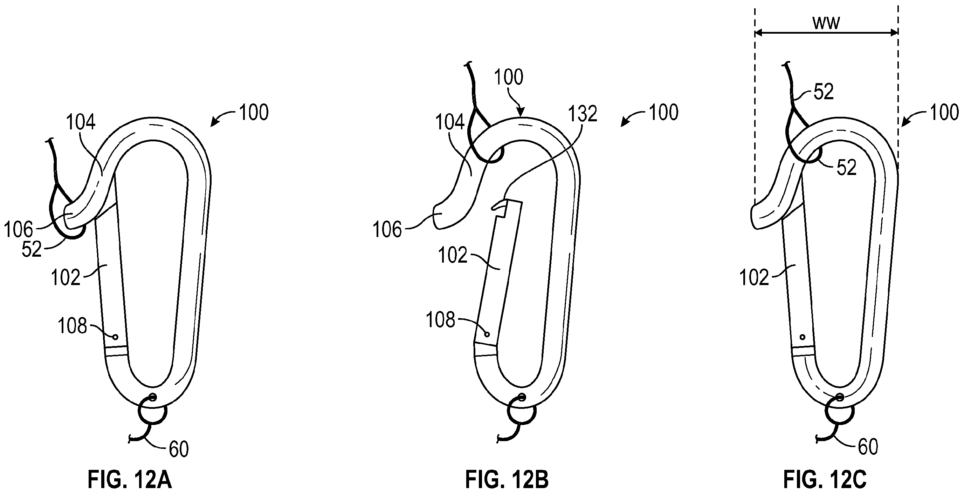

Turning To Figs. 12A to 12C, another technique for attaching a gastrointestinal implant 40 to an implanted tissue anchor 54A or anchor pair 50 includes using a clasp 100 . The clasp 100 may easily attach to a loop 52, while also providing a reliable attachment. The clasp 100 may have a generally oval shape with a resilient or spring loaded rotating arm 102. The arm 102 rotates inwardly when an external opening force is applied, to receive the loop 52. The arm 102 springs back into the closed position shown in Figs. 12A and 12C after the opening force is removed. The clasp 100 has an overhang 104 to prevent the arm 102 from rotating outwardly, which prevents the loop 52 from slipping off of the clasp 100. The upper free end of the arm 102 may have a step 132 which moves into a slot in the overhang 104. The clasp 100 may be oval shaped. The clasp has a maximum width WW of 2 to 4 or 1.8 to 2.6 millimeters and a length of 1.5 to 2.4 WW.

The clasp 100 may include an extension 106 adjacent to the arm 102, opposite from the arm pivot joint 108, for hooking the loop 52 of the anchor pair 50. With this design a simple pull will deflect the arm 102 inwardly allowing the loop 52 to move into the clasp 100 and be captured by the clasp 100.

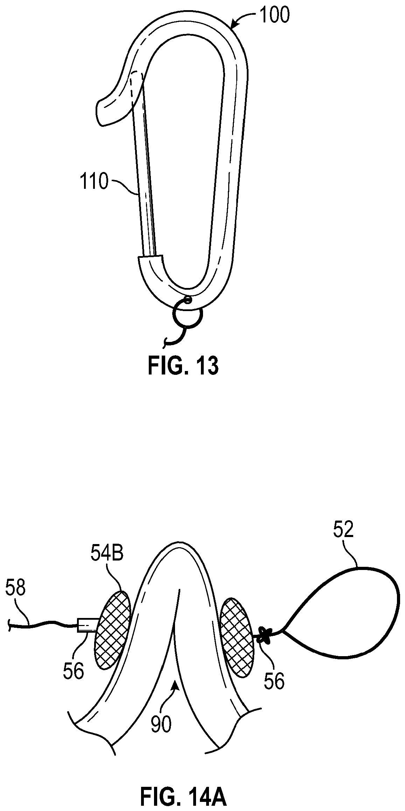

In an alternative design shown in Fig. 13 the arm may be a flex arm 110, rather than a rotating arm 102. In this design, a wire such as a Nitinol or stainless steel wire, or other flex element may be used as the flex arm 110. The flex arm 110 deflects inwardly sufficiently to allow the loop 52 to pass into the clasp 100, and the arm 110 then springs back outwardly. The arm 102 or 110 may be straight when in the closed position shown in Fig. 13. In a similar design, the entire clasp, including the arm, may be a plastic or non-metal, for example provided as a single molded plastic component. Although the clasp 100, if used, is generally attached to or made part of the implant 40, for some applications the clasp 100 may alternatively be attached to or made part of the tissue anchor pair 50.

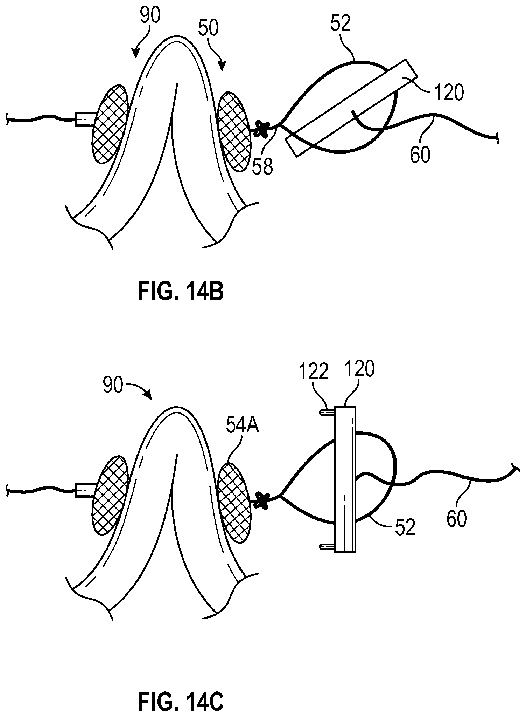

A gastrointestinal implant 40 may alternatively be attached to an implanted anchor pair 50 using a tee bar 120 and a loop 52. Referring to Figs. 14A-14C, the GI implant 40 includes a tee bar 120 at one end. The tee bar 120 is generally positioned perpendicular to the axis of the tether 60, in its undeflected position, but can deflect into a generally parallel position for passing through the loop 52.

The length of the tee bar 120 and the loop 52 are sized to allow passage of the tee bar 120 through the loop 52, but also preventing removal of the tee bar from the loop after it is placed. Additional features may be provided on the tee bar 120 and/or on the loop 52 to keep the tee bar generally perpendicular after it is inserted through the loop.

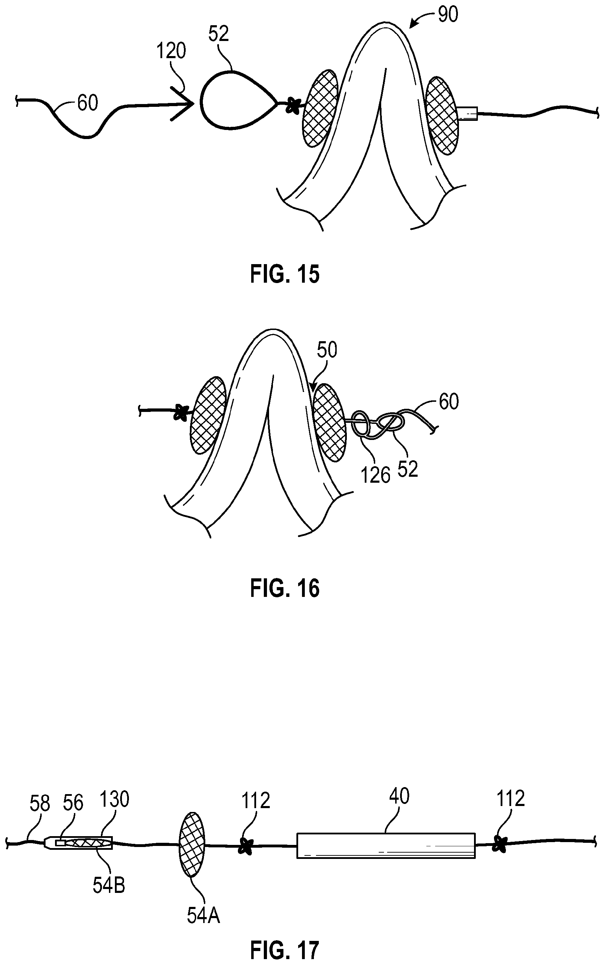

Tee bar embodiments may have other forms to provide additional benefits. For example, the tee bar 120 may have protrusions 122 at the ends of the tee bar to further prevent the loop 52 from inadvertently slipping off of the tee bar 120. The tee bar 120 may also be V-shaped as shown in Fig. 15 or cone shaped to both assist in insertion through the loop and to better prevent slipping out of the loop 52. Tee bar embodiments may be loaded into a catheter 46 while in a low profile position and deployed or opened after inserting the catheter tip through the loop 52.

A loop to loop attachment may also be used to attach a gastrointestinal implant 40 to an implanted anchor pair 50, as shown in Fig. 16. In this design, the gastrointestinal implant 40 has an implant loop 126 at one end. The implant loop 126 may be made of braided polyester suture. The anchor pair 50 also has an anchor pair loop 52, which may be made of the same material. The gastrointestinal implant 40 is attached to the anchor pair 50 by coupling or interlocking the two loops together. This may be achieved by passing the gastrointestinal implant loop 126 over the anchor pair loop 52 and then routing the other end of the gastrointestinal implant 40 through the anchor pair loop 52.

An anchor pair may also be integrated directly into a gastrointestinal implant. For example, Fig. 17 shows an implant anchor assembly 128 which includes a gastrointestinal implant 40 pre-connected to an anchor pair 50. A knot 112 is positioned between the implant 40 and the first tissue anchor 54A, which is in the expanded or deployed position. As shown in Fig. 17, a second tissue anchor 54B is positioned inside of a tube 130 in the unexpanded configuration. A suture extension 58A is attached to the implant 40 and extends through the first and second tissue anchors 54A and 54B and a cinch 56. The cinch 56 and the second anchor 54B are inside of the tube 130, which may have a sloped or conical front end 131. With the second anchor 54B compressed inside of the tube 130, the suture extension 58A cannot slide freely through the tube 130. A knot 112 is provided in the suture extension 58A between the first tissue anchor 54A and the implant 40.

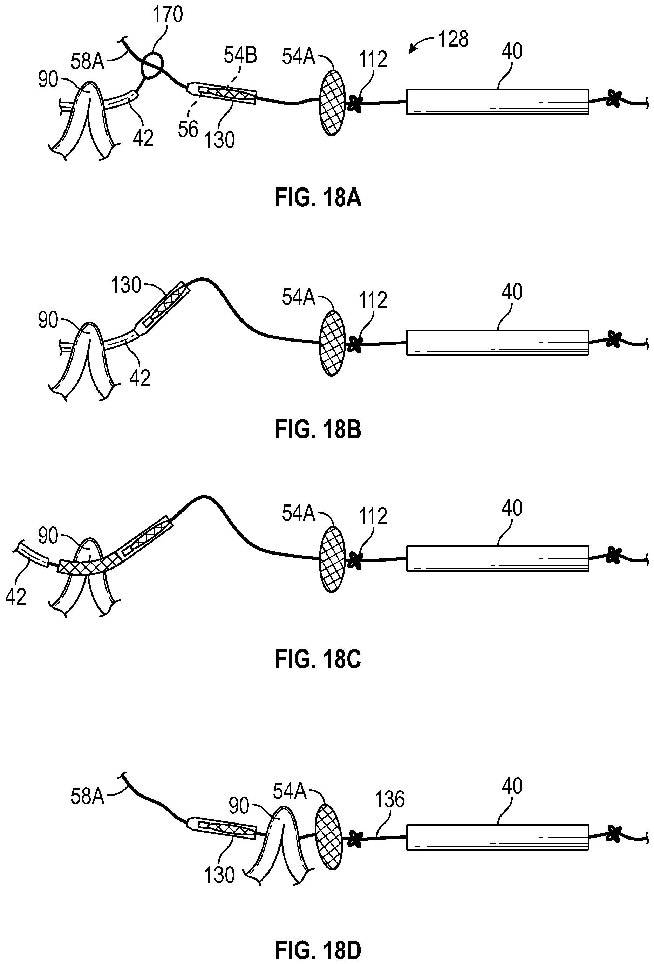

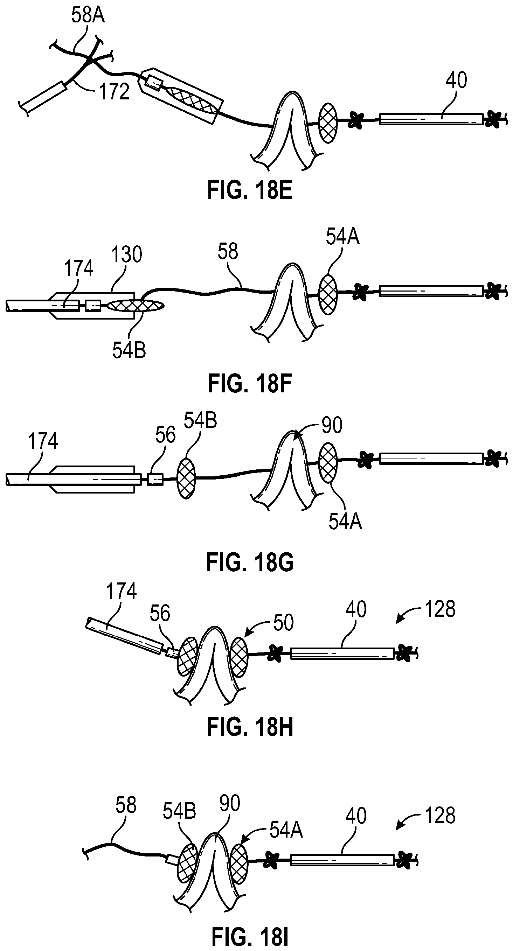

Turning to Figs. 18A to 18D, the implant anchor assembly 128 is pushed out of delivery device, such as via an endoscope channel. The implant 40 may be positioned as desired using endoscopic techniques. If the implant has any of its own anchoring devices, these may be deployed or engaged. In Figs. 18A-D the anchor pair 50 is delivered onto the tissue fold 90 by pulling the tube 130 through the tissue fold 90. As shown in Fig 18A, the tube 130 is on the first side of the tissue fold 90. The tip of a hypodermic needle 42 is pushed through a tissue fold 90. A loop or snare 170, such as a spring wire snare, is then pushed out of the needle 42. The snare is maneuvered around the suture extension 58A attached to the implant 40, optionally with the assistance of a separate grasping tool. Alternatively, a grasping tool 172, instead of a snare 170, may be deployed out from the end of the tip of the needle, with the grasping tool grasping the suture extension 58A, as shown in Fig. 18E.

The snared or grasped suture extension 58A is then retracted into needle 42, drawing the front end of the tube 130 adjacent to or into contact with the tip of the needle 42, as shown in Fig 18B. The needle 42 and the tube 130 are then pulled back through the tissue fold 90, as shown in Fig 18C. After the tube 130 is pulled to the second side of the tissue fold 90, the snare 170 or grasping tool is released from the suture extension 58A of the implant 40. The expanded anchor 54A is left on the first side of the tissue fold, as shown in Fig 18D.

As shown in Fig. 18F, after the tube 130 is pulled through to the second side of the tissue fold 90, the second anchor 54B is deployed. This may be achieved by pushing a pusher 174 out of the needle 42 and into the tube 130. The tube 130 may be held in place via the snare 179 or grasping tool 172 holding the tube or the suture extension 58A. Alternatively, the tube may be temporarily held in place by a separate endoscopic tool. Fig. 18G shows the second tissue anchor 54B now deployed and in the expanded configuration, with the cinch 56 also pushed out of the tube 130. Fig. 18H shows the pusher pushing the cinch 56 and the second tissue anchor 54B up against the second side of the tissue fold 90. Fig. 18I shows the implant 40 now secured to the tissue fold 90 via the tissue anchor pair 50. The snare or grasper is released from the suture extension 58A of implant 40. The tube 130 is secured onto the suture extension 58A or is removed via a lumen in an endoscope or delivery catheter.

In Figs. 18A-18G the implant 40 is shown as a rod of material or several rods of material chained or connected together, with the rods loaded with microbiota. As described here 54A indicates the first tissue anchor deployed and 54B indicates a second or subsequent tissue anchor deployed. Also as used here, suture indicates conventional surgical suture as well as equivalents such as metal or non-metal fibers or filaments.

As shown in Figs. 19 and 20, a dual clasp assembly 140 has first and second clasps with suture 58 or wire attached to the first clasp and running through a clearance hole in the second clasp. A cinch 56 is slidably positioned on the suture 58. The clasps may be any of the clasps described herein. In use, the first clasp is attached to an anchor point or loop 142 on the gastrointestinal implant 40. The second clasp is attached to the loop 52 of the anchor pair 50. Multiple dual clasp assemblies may be attached to anchor points 142 on the gastrointestinal implant 40 and to multiple loops 52 of tissue anchor pairs 50. The cinch 56 may be moved along the suture to adjust the length of the suture 58 between the clasps. The cinch 56 resists movement in the reverse direction, which prevents the clasps from moving apart after the cinch is used to adjust the suture length. After the clasps are attached to the gastrointestinal implant and to the anchor pair(s), the cinch 56 may be advanced along the suture, shortening the maximum allowable distance between the clasps. This may be used to remove unwanted slack in the suture, for optimizing the spacing between the implant and the anchor pair, for optimizing the position of the implant relative to surrounding tissue, or for providing consistent tension along separate sutures, if more than one suture or attachment point is used. The dual clasp assembly 140 may be made small enough to be loaded into a catheter for delivery during use. The adjustable length suture with the cinch may be attached inside the catheter in a way to allow the user to easily cinch the suture. This avoids the need for grasping the suture with a secondary device.

In some applications the suture may be replaced with an elastic material to compensate for movement of tissue within the gastrointestinal tract, such as with peristalsis.

Fig. 21 shows a clasp 100 similar to Fig. 13 but having an oval shape.

Fig. 22 shows a clasp 100 similar to Fig. 21 but having a second flex arm to further secure a loop 52 within the clasp.

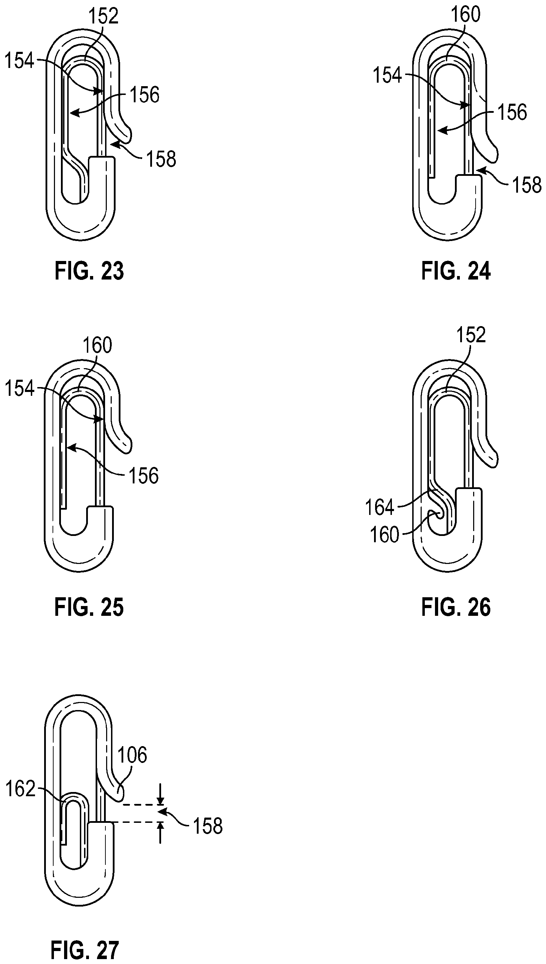

Fig. 23 shows a clasp similar to Fig. 21 but having a shortened opening 158 and a hook-shaped insert 152 which creates first and second pinch points 154 and 156. The shortened opening 158 may be 10 to 30% of the total length of the clasp, whereas the longer opening OP shown in Fig. 21 may be 45 to 80% of the total length of the clasp. Fig. 24 shows a similar design having a U-shaped insert 160. Fig. 25 shows a clasp which may be the same as Fig. 24 but having the longer opening OP. Fig. 26 shows a design using the hook-shaped insert 152 and further including a projection 160 contacting the diagonal section 164 of the insert 152. Fig. 27 shows a design similar to Fig. 21 but including a U-shaped insert 162 extending from the bottom of the opening of the clasp up to only the opening 158 or to the extension 106.

In the following paragraphs, methods useful in connection with the present invention are described. These methods do not form part of the scope of protection of the present invention.

Methods may include forming a first tissue fold; piercing the first tissue fold with a needle; deploying a first tissue anchor from the needle; withdrawing the needle from the first tissue fold; deploying a second tissue anchor from the needle, with the first tissue anchor connected to the second tissue anchor to form a first tissue anchor pair; and attaching a gastrointestinal implant to the tissue anchor pair. The gastrointestinal implant may have a tether including a tether loop, the methods further including attaching the gastrointestinal implant to the tissue anchor pair by positioning the tether loop between the tissue fold and first anchor. The tether loop may be positioned between the tissue fold and the second anchor. Advancing a cinch towards the second tissue anchor can prevent the second tissue anchor from moving away from the first tissue anchor.

The gastrointestinal implant may be a gastric sleeve in the duodenum and the tissue fold in the antrum. A needle may be extended through the tether loop before deploying the first tissue anchor.

The methods may further include forming a second tissue fold, piercing the second tissue fold with the needle, deploying a third tissue anchor from the needle, withdrawing the needle from the third tissue fold, deploying a fourth tissue anchor from the needle, with the third tissue anchor connected to the fourth tissue anchor to form a second tissue anchor pair; and attaching the gastrointestinal implant to the first tissue anchor pair and to the second tissue anchor pair.

The first and second tissue anchor pairs may each have an anchor loop and the attaching step is performed by routing a tether loop attached to the gastrointestinal implant through the anchor loops. The gastrointestinal implant may have a first tether and a second tether, further including attaching the first tether to the first tissue anchor pair and attaching the second tether to the second tissue anchor pair. The tether loop may be suture or wire formed into a loop.

The gastrointestinal implant may include a ring attached directly or indirectly to the first tissue anchor pair. The first tissue anchor may be positioned inside of the gastrointestinal implant.

The first and third tissue anchors can be positioned inside of the gastrointestinal implant. The first and second tissue folds may be at least partially positioned inside of the gastrointestinal implant.

The gastrointestinal implant may have an attachment element such as a prong, a hook or a protrusion. In this case the method may include moving the first tissue anchor loop into engagement with the attachment element. The tether loop may have for grasping with a grasping tool.

In an alternative method, a tee bar attached to a tether of the implant is moved through a tissue anchor loop. The tee bar may have a V-shape or a conical shape.

In cases where the first tissue anchor pair has a tissue anchor loop, and the gastrointestinal implant has a tether loop, they may be attached by interlocking the tissue anchor loop with the tether loop.

The gastrointestinal implant may be provided as a rod of material or several rods of material connected together, with the rods loaded with microbiota.

An implant anchor assembly includes a gastrointestinal implant attached to an extension, which may be a suture extension. The suture extension passes through a first tissue anchor in an expanded or deployed configuration. A know may be provided in the suture extension between the first tissue anchor and the gastrointestinal implant. The suture extension also extends through a second tissue anchor and a cinch, which are in a tube. The second tissue anchor is compressed within the tube, and in an un-extended or undeployed configuration. The suture extension may be wedged or clamped in the tube so that tube cannot slide along the suture extension until after the second tissue anchor is deployed from the tube. The tube may have a sloped or conical front end.

In a method of using the implant anchor assembly, the tube containing the second tissue anchor and the cinch is pulled through a tissue fold. The second tissue anchor and the cinch are then deployed out of the tube and advanced up against the tissue fold.

## Claims

1. Surgical apparatus, having a needle (42) containing a first tissue anchor (54A) deployable out from the needle (42) after piercing a first tissue fold, the needle (42) also containing a second tissue anchor (54B) connected to the first tissue anchor (54A) to form a tissue anchor pair (50) having a tissue anchor pair loop (52);
and
a tether loop (62) attached on a tether (60);
**characterized by**:
a gastrointestinal implant (40) attached to the tether (60); and
a clasp (100) having a movable arm (102), the clasp (100) attached to the tether loop on the gastrointestinal implant (40), the tissue anchor pair loop (52) engageable with the clasp (100) by moving the tissue anchor pair loop (52) onto the clasp (100) to attach the gastrointestinal implant (40) to the tissue anchor pair (50).

2. The apparatus of claim 1 wherein the arm (102) is pivotally attached onto the clasp (100) and is spring loaded into a closed position.

3. The apparatus of claim 1 wherein the arm (110) is flexible to allow the arm (110) to be temporarily displaced by bending the arm (110).

4. The apparatus of any one of claims 1-3 further including a second clasp (100) engageable with the gastrointestinal implant (40), with the clasp (100) and the second clasp (100) linked via suture or wire (58).

5. The apparatus of claim 4 further including a cinch (56) movable along the suture or wire (58) in only one direction, for adjusting a length of the suture or wire (58).

6. The apparatus of any one of claims 1-5 further including an extension (106) projecting outwardly from the clasp (100) for hooking the tissue anchor pair loop (52) onto the clasp (100).

7. The apparatus of claim 1 further including a tab (66) on the tether loop (62).

8. The apparatus of claim 1 wherein the gastrointestinal implant (40) is a gastrointestinal sleeve.

## Patentansprüche

1. Chirurgische Vorrichtung, mit einer Nadel (42), welche einen ersten Gewebe-Anker (54A) aufweist, welcher aus der Nadel (42) heraus entfaltbar ist, nachdem eine erste Gewebefalte durchstochen wurde, wobei die Nadel (42) weiter einen zweiten Gewebe-Anker (54B) beherbergt, welcher mit dem ersten Gewebe-Anker (54A) verbunden ist, um es so ein Gewebe-Ankerpaar (50) auszubilden, welches eine Gewebe-Ankerpaar-Schlaufe (52) und eine Halteleinen-Schlaufe (62) aufweist, an welcher einer Halteleine (60) angebracht ist;
**gekennzeichnet durch**
ein Magen-Darm-Implantat (40), welches an der Halteleine (60) befestigt ist; und
ein Verschlusselement (100), welches einen bewegbaren Arm (102) aufweist, wobei das Verschlusselement (100) an der Halteleinen-Schlaufe auf dem Magen-Darm-Implantat (40) befestigt ist, wobei die Gewebe-Ankerpaar-Schlaufe (52) mit dem Verschlusselement (100) in Eingriff bringbar ist, indem die Gewebe-Ankerpaar-Schlaufe (52) auf das Verschlusselement (100) bewegt wird, um so das Magen-Darm-Implantat (40) an dem Gewebe-Ankerpaar (50) zu befestigen.

2. Die Vorrichtung nach Anspruch 1, wobei der Arm (102) schwenkbar an dem Verschlusselement (100) angebracht ist und mit einer Feder in eine geschlossene Position vorgespannt ist.

3. Die Vorrichtung nach Anspruch 1, wobei der Arm (110) flexibel ist und so erlaubt, den Arm (110) vorübergehend durch ein Verbiegen des Arms (110) zu verlagern.

4. Die Vorrichtung nach einem der Ansprüche 1-3, weiter versehen mit einem zweiten Verschlusselement (100), welches mit dem Magen-Darm-Implantat (40) in Eingriff bringbar ist, wobei das Verschlusselement (100) und das zweite Verschlusselement (100) mittels eines Fadens oder eines Drahts (58) miteinander verbunden sind.

5. Die Vorrichtung nach Anspruch 4, weiter versehen mit einem Stoppelement (56), welches entlang des Fadens oder Drahts (58) in nur einer Richtung verschiebbar ist, um eine Länge des Fadens oder Drahts (58) einzustellen.

6. Die Vorrichtung nach einem der Ansprüche 1-5, weiter versehen mit einer Verlängerung (106), welche sich ausgehend von dem Verschlusselement (100) nach außen erstreckt, um die Gewebe-Ankerpaar-Schlaufe (52) auf das Verschlusselement (100) zu haken.

7. Die Vorrichtung nach Anspruch 4, weiter versehen mit einer an der Halteleinen-Schlaufe (62) angebrachten Lasche (66).

8. Die Vorrichtung nach Anspruch 1, wobei das Magen-Darm-Implantat (40) eine Magen-Darm-Hülle ist.

## Revendications

1. Appareil chirurgical, ayant une aiguille (42) contenant un premier ancrage tissulaire (54A) déployable hors de l'aiguille (42) après avoir percé un premier pli tissulaire, l'aiguille (42) contenant également un second ancrage tissulaire (54B) connecté au premier ancrage tissulaire (54A) pour former une paire d'ancrages tissulaires (50) ayant une boucle de paire d'ancrages tissulaires (52) ; et
une boucle d'attache (62) fixée à une attache (60) ; **caractérisé par** :
un implant gastro-intestinal (40) fixé à l'attache (60) ; et
un fermoir (100) ayant un bras mobile (102), le fermoir (100) étant fixé à la boucle d'attache sur l'implant gastrointestinal (40), la boucle de paire d'ancrages tissulaires (52) pouvant venir en prise avec le fermoir (100) en déplaçant la boucle de paire d'ancrages tissulaires (52) sur le fermoir (100) pour fixer l'implant gastro-intestinal (40) à la paire d'ancrages tissulaires (50).

2. Appareil selon la revendication 1, dans lequel le bras (102) est fixé de manière pivotante sur le fermoir (100) et est chargé par ressort dans une position fermée.

3. Appareil selon la revendication 1, dans lequel le bras (110) est flexible pour permettre au bras (110) d'être temporairement déplacé en pliant le bras (110).

4. Appareil selon l'une quelconque des revendications 1 à 3, comprenant en outre un second fermoir (100) pouvant venir en prise avec l'implant gastro-intestinal (40), le fermoir (100) et le second fermoir (100) étant reliés via une suture ou un fil (58).

5. Appareil selon la revendication 4, comprenant en outre une sangle (56) mobile le long de la suture ou du fil (58) dans une seule direction, pour ajuster une longueur de la suture ou du fil (58).

6. Appareil selon l'une quelconque des revendications 1 à 5, comprenant en outre une extension (106) faisant saillie vers l'extérieur depuis le fermoir (100) pour accrocher la boucle de paire d'ancrages tissulaires (52) sur le fermoir (100).

7. Appareil selon la revendication 1, comprenant en outre une languette (66) sur la boucle d'attache (62).

8. Appareil selon la revendication 1, dans lequel l'implant gastro-intestinal (40) est un manchon gastro-intestinal.
